# EUROPEAN PATENT APPLICATION

(11) **EP 2 886 048 A1**
(43) Date of publication of application: **24.06.2015**
(21) Application number: 13879491.2
(22) Date of filing: 13.08.2013
(51) Int. Cl.: A61B 5/02, A61B 5/145

(54) **REAL-TIME BIOSIGNAL MEASUREMENT APPARATUS FOR CARDIAC ISCHEMIA AND REPERFUSION**

(30) Priority: 14.08.2012 KR 20120089089
(71) Applicant: University-Industry Cooperation Group of Kyung Hee University, Gyeonggi-do 446-701 (KR)
(72) Inventor: PARK, Hun Kuk, Seoul 130-820 (KR); LEE, Gi Ja, Seoul 139-956 (KR); KIM, Ok Kyun, Seoul 130-820 (KR); KANG, Sung Wook, Seoul 142-812 (KR)
(74) Representative: Pallini Gervasi, Diego
(86) International application number: PCT/KR2013/007277
(87) International publication number: WO 2014/027820

(57) **Abstract**

The present invention relates to a real-time biosignal measurement apparatus for cardiac ischemia and reperfusion. The apparatus includes: a peristaltic pump which pumps perfusate from a tank storing the perfusate; a pressure trap which offsets the pulsation of both sides between a solution chamber temporarily storing the pumped perfusate and the perfusate tank; a heat exchanger to which the temporarily stored perfusate is applied and which transfers the heat of the perfusate; a bubble trap to which the heat transferred perfusate is applied on one side and which filters the heat transferred perfusate to transduce the perfusate to an isolated heart connected to the other side; a sensor unit which detects the gas concentration value in the myocardium of the isolated heart in real time during the cardiac ischemia reperfusion; and a control unit which is supplied with the gas concentration value in the myocardium of the isolated heart in order to analyze its correlation with the degree of a myocardial injury of the isolated heart.

## Description

### TECHNICAL FIELD

The present invention relates to a real-time biosignal measurement apparatus, and more particularly, to a real-time biosignal measurement apparatus for cardiac ischemia and reperfusion that is capable of precisely monitoring a gasotransmitter and myocardial oxygen concentration in real time that is significant in evaluating myocardium survival capacity and blood vessel function caused by ischemia-reperfusion (IR) injury.

### BACKGROUND ART

In general, despite improvements in clinical disease management of acute myocardial infarction (AMI), (and as one of the leading causes of morbidity and death in advanced nations, AMI is a serious disease with a 40% mortality rate upon occurrence.)

AMI occurs when blood vessels supplying oxygen and nutrients to the myocardium are clogged, and the myocardium is injured because it is not receiving enough oxygen. It is significant to carry out reperfusion within a shortest time and to re-supply oxygen and nutrients to the myocardium.

However, reperfusion itself is known to cause injury. Reactive oxygen species (ROS), a leading result of reperfusion injury, is produced mostly from the mitochondrial respiratory chain under pathological conditions including ischemia-reperfusion (IR).

In particular, nitric oxide (NO) is known to play a significant role in cardiovascular system by adjusting various pathophysiological procedures, is especially associated with controlling vascular homeostasis and has a protective effect against ischemic cell death.

NO is known to not only suppresses oxidative stress and cytokine release, but the small amount of NO synthesized by constitutive nitric oxide synthase (NOS) also regulates blood flow to the coronary artery, as well as myocardial oxygen consumption.

Thus, precise measurement of endogenous NO concentration is positively necessary to understand a biological role and a signal transduction procedure of NO. However, NO exists in the body to have a nano-molar concentration, has a very short half-life period of about 2 to 6 seconds, reacts very strongly with many intrinsic substances, such as free radical, peroxides, and oxygen and therefore, a limit to precise measurement of NO concentration using temporal and spatial resolution.

According to recent research, the amount of oxygen transported to the myocardium during reperfusion is the main determining factor in heart failure after ischemia and IR damage. In this way, the state of the myocardium is very significant so that the heart functions normally. Thus, precise measurement of the oxygen concentration in the myocardium after the ischemic period is very significant to evaluate a myocardium survival capacity and function caused by the IR injury.

To this end, in the related art, a fluid introduced into or discharged from the reperfused heart is used to measure an oxygen consumption amount in the myocardium of an experiment rodent. This method has problems that the concentration of oxygen cannot be measured in real time during a no-flow ischemic period and tissue heterogeneity in oxygen transfer or metabolism cannot be calculated.

Also, oxygen measurement methods based on a particle spin probe and electron paramagnetic resonance (EPR) may react quite sensitively when measuring myocardial oxygen consumption, but is problematic due to its inability to measure continuous real-time variations in oxygen levels within the concentration range of several mmHg to several hundreds of mmHg.

### DETAILED DESCRIPTION OF THE PRESENT INVENTION

### TECHNICAL PROBLEM

An objective of the present invention is to provide a real-time biosignal measurement apparatus for cardiac ischemia and reperfusion, which can evaluate the degree of damage in cardiac ischemia and reperfusion, by measuring the amount of variation in endothelium-dependent vasoactive gasotransmitter intrinsically generated in the myocardium during an ischemic period using an oxygen sensor and quantitatively analyzing the degree of reoxygenation of the myocardium during reperfusion while monitoring whether an isolated heart from rat is maintained in living state and cardiac ischemia occurs and reperfusion is normally performed using an oxygen sensor.

### TECHNICAL SOLUTION

According to an aspect of the present invention, there is provided a real-time biosignal measurement apparatus for cardiac ischemia and reperfusion including: a peristaltic pump which pumps perfusate from a tank storing the perfusate; a pressure trap which offsets the pulsation of both sides between a solution chamber temporarily storing the pumped perfusate and the perfusate tank; a heat exchanger to which the temporarily stored perfusate is applied and which transfers the heat of the perfusate; a bubble trap to which the heat transferred perfusate is applied on one side and which filters the heat transferred perfusate to transduce the perfusate to an isolated heart connected to the other side; a sensor unit which detects the gas concentration value in the myocardium of heart in real time during the cardiac ischemia reperfusion; and a control unit which is supplied with the gas concentration value in the myocardium of the isolated heart in order to analyze its correlation with the degree of a myocardial injury of the heart.

In order to accomplish the above objective, the present real-time biosignal measurement apparatus for cardiac ischemia and reperfusion analyzes the correlation using an oxygen concentration restored after reperfusion for a predetermined time after ischemia occurs and a maximum oxygen concentration during reperfusion.

In order to accomplish the above objective, the present real-time biosignal measurement apparatus for cardiac ischemia and reperfusion evaluates the degree of a myocardial injury by the size of myocardial infarction of the isolated heart according to a variation in the ischemic period.

In order to accomplish the above objective, the present real-time biosignal measurement apparatus for cardiac ischemia and reperfusion indicates the size of myocardial infarction by a percentage of a risk territory by staining with triphenyltetrazolium chloride (TTC).

In order to accomplish the above objective, the present real-time biosignal measurement apparatus for cardiac ischemia and reperfusion sets a predetermined time ranging from 55 to 65 minutes.

In order to accomplish the above objective, the present real-time biosignal measurement apparatus for cardiac ischemia and reperfusion includes at least one of the following: an oxygen sensor that detects an oxygen concentration in the myocardium of the isolated heart in real time; a nitric oxide (NO) sensor that detects an NO concentration in the myocardium of the isolated heart in real time; a carbon oxide (CO) sensor that detects a CO concentration in the myocardium of the isolated heart in real time; and a hydrogen sulfide sensor that detects a hydrogen sulfide concentration in the myocardium of the isolated heart in real time.

In order to accomplish the above objective, the present real-time biosignal measurement apparatus for cardiac ischemia and reperfusion measures the oxygen concentration reduced when cardiac ischemia occurs, the oxygen concentration restored after reperfusion, and the maximum oxygen concentration during reperfusion using the oxygen sensor and may measure a variation in the NO concentration during the ischemic period and initial decrement and maximum increment of NO concentration during ischemia and reperfusion, thereby detecting the gas concentration value.

In order to accomplish the above objective, the present real-time biosignal measurement apparatus for cardiac ischemia and reperfusion further includes a potentiostat that supplies a constant electric potential to the sensor unit and measures a variation in current.

In order to accomplish the above objective, the present real-time biosignal measurement apparatus for cardiac ischemia and reperfusion further includes the heat exchanger: a thermocouple that is immediately in contact with a part to be connected to the aorta of the isolated heart; and a temperature controller that maintains a temperature of the perfusate flowing into the isolated heart at a constant temperature.

In order to accomplish the above objective, the present real-time biosignal measurement apparatus for cardiac ischemia and reperfusion maintains a predetermined temperature ranging from 35 °C to 39 °C.

In order to accomplish the above objective, the present real-time biosignal measurement apparatus for cardiac ischemia and reperfusion maintains the solution chamber at a predetermined height with the isolated heart.

In order to accomplish the above objective, the present real-time biosignal measurement apparatus for cardiac ischemia and reperfusion has height ranging from 85 to 95 cm.

### EFFECTS OF THE INVENTION

According to the present invention, the pressure of perfusate to be transduced to heart blood vessels for supplying oxygen and nutrients to the myocardium of the isolated heart using a pressure trap that offsets the pulsation of both sides can be maintained at a constant level.

In addition, the concentration of oxygen in the myocardium can be continuously measured in real time using an oxygen sensor having a low operation potential and high sensitivity.

In addition, continuous real-time measurement of the change in gasotransmitter concentration in the myocardium can be performed using a gasotransmitter sensor having high sensitivity and excellent selectivity.

Furthermore, the correlation between the degree of an ischemia-reperfusion (IR) injury and a gas concentration value of the isolated heart can be precisely analyzed, and the present invention can be usefully utilized to evaluate and select various drugs and treatments that may reinforce an endogenous protective mechanism against an injury.

### DESCRIPTION OF THE DRAWINGS

FIG. 1 is a configuration view and a partially-enlarged view of a real-time biosignal measurement apparatus for cardiac ischemia and reperfusion according to the present invention.
FIG. 2 is a graph showing representative patterns of an oxygen concentration (pO₂) response during cardiac ischemia reperfusion according to an ischemic period according to the present invention.
FIGS. 3A and 3B are graphs showing the definition of suggested analysis parameters with respect to a variation in the oxygen concentration according to an experimental protocol according to the present invention.
FIG. 4 is a Box-and-Whisker graph showing maximum and restoration levels of the oxygen concentration during reperfusion that are percentages of pre-ischemic period levels according to the ischemic period according to the present invention.
FIG. 5 is a graph showing the sizes of myocardial infarction in the heart of rat according to a variation in the ischemic period according to the present invention.
FIG. 6 are photos comparing the heart of rat, the sizes of the myocardial infarction changing according to a variation in the ischemic period according to the present invention.
FIG. 7 is a graph showing the correlation between reoxygenation parameters of the myocardium after the ischemic period and the degree of severity of an ischemia-reperfusion (IR) injury according to the present invention.
FIG. 8 is a graph showing the result of simultaneously measured variations of nitric oxide (NO) and oxygen measured in the myocardium of rat during the ischemic period of 30 minutes and reperfusion for 60 minutes according to the present invention.

### BEST MODE

Hereinafter, a real-time biosignal measurement apparatus for cardiac ischemia and reperfusion according to an exemplary embodiment of the present invention will be described with the accompanying drawings.

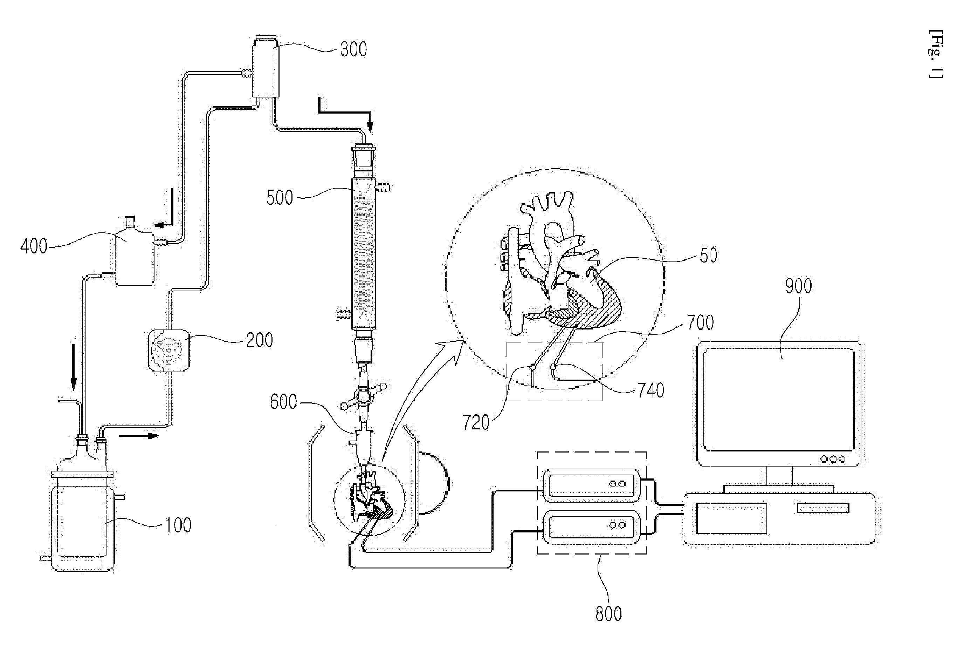

FIG. 1 is a configuration view and a partially-enlarged view of a real-time biosignal measurement apparatus for cardiac ischemia and reperfusion according to the present invention. The real-time biosignal measurement apparatus for cardiac ischemia and reperfusion according to the present invention includes a perfusate tank 100, a peristaltic pump 200, a solution chamber 300, a pressure trap 400, a heat exchanger 500, a bubble trap 600, a sensor unit 700, a potentiostat 800, and a control unit 900. The sensor unit 700 includes an oxygen sensor 720 and a nitric oxide (NO) sensor 740.

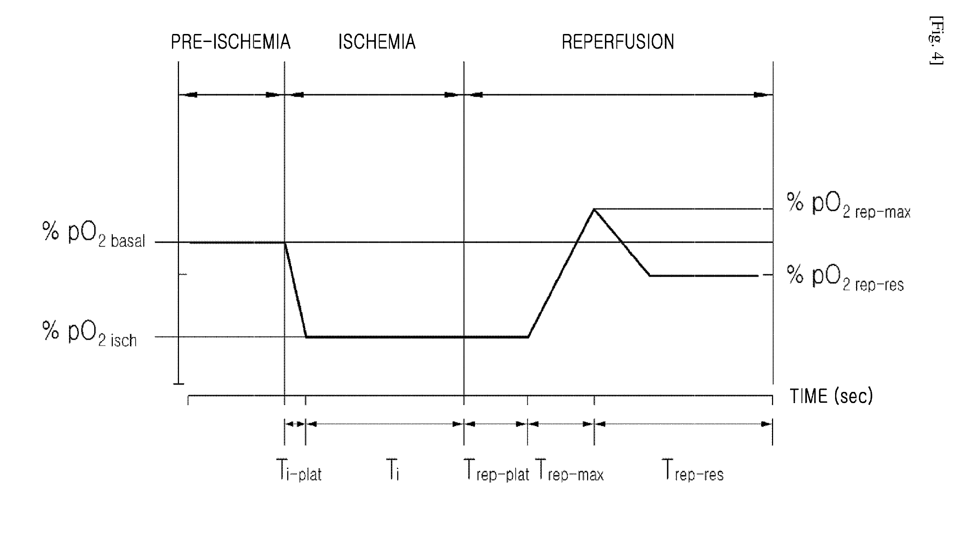

FIG. 4 is a graph showing the size of the myocardial infarction portion of rat according to a variation in an ischemic period according to the present invention.

Functions of the elements of the real-time biosignal measurement apparatus for cardiac ischemia and reperfusion according to the present invention will now be described with reference to FIG. 1.

The perfusate tank 100 stores perfusate. A Krebs-Henseleit (K-H) solution in which 95% oxygen and 5% carbon dioxide are dissolved, is used as the perfusate.

The peristaltic pump 200 pumps the perfusate from the perfusate tank 100 and transduces the pumped perfusate to the solution chamber 300.

The solution chamber 300 temporarily stores the perfusate pumped while maintaining a constant height with an isolated heart 50.

The pressure trap 400 offsets the pulsation of both sides between the solution chamber 300 and the perfusate tank 100.

The heat exchanger 500 having one side to which the temporarily stored perfusate is applied from the solution chamber 300, transfers the heat of the perfusate and maintains the temperature of the perfusate at a constant level.

The bubble trap 600 having one side to which the perfusate tranduced after passing through the heat exchanger 500 is applied, filters the perfusate and transduces the filtered perfusate to the isolated heart 50 connected to the other side.

The sensor unit 700 detects a gas concentration value of the isolated heart 50 in real time during the cardiac ischemia reperfusion. That is, the oxygen sensor 720 detects an oxygen concentration in the myocardium of the isolated heart 50 in real time, and the NO sensor detects an NO concentration in the myocardium of the isolated heart 50 in real time.

In this case, the sensor unit 700 may include a plurality of carbon oxide (CO) sensors and a plurality of hydrogen sulfide sensors in addition to the oxygen sensor 720 and the NO sensor 740.

The potentiostat 800 supplies a constant electric potential to the sensor unit 700 and measures the variation in current caused thereby.

The control unit 900 observes a variation in the detected gas concentration in real time and analyzes its correlation with the degree of a myocardial injury of the isolated heart 50 to which the variation in the gas concentration is applied. That is, the control unit 900 analyzes the correlation between the detected gas concentration value using an oxygen concentration restored after reperfusion for a predetermined amount of time after ischemia occurs and a maximum oxygen concentration during reperfusion and the degree of a myocardial injury of the isolated heart 50.

An operation of the real-time biosignal measurement apparatus for cardiac ischemia and reperfusion according to the present invention will now be described with reference to FIGS. 1 and 4.

First, a thermocouple is immediately in contact with a part to be connected to the aorta that flows into the heart 50 using the perfusate tank 100 having a double jacket shape and the heat exchanger 500, and the temperature of the perfusate that flows into the heart 50 is maintained at 35 °C to 39 °C, preferably, at 37 °C using a temperature controller that is self-produced.

This is a normal body temperature of an experiment mouse for optimizing experiments. If the temperature of the perfusate is less than 35 °C, there is a possibility that isolated heart of rat will be dead due to hypothermia, and if the temperature of the perfusate exceeds 39 °C, degeneration of protein or an injury of cells may occur.

Also, in order to tranduce the perfusate to the heart blood vessels for supplying oxygen and nutrients to the myocardium, the solution chamber 300 is maintained at a constant height of 85 to 95 cm, preferably, 90 cm with the isolated heart and at a constant perfusate pressure of 60 to 70 mmHg, preferably, 65 mmHg using the pressure trap 400.

This is because, if the height is less than 85 cm, the ostial flap cannot be bent back and thus, there is a possibility that the perfusate will not be transduced to the heart blood vessels, and if the height exceeds 95 cm, there is a possibility that the perfusate will be transduced to the atrium (not to the heart blood vessels).

In this case, the peristaltic pump 200 pumps the perfusate from the perfusate tank 100 storing the perfusate, and the pressure trap 400 offsets the pulsation of the solution chamber 300 temporarily storing the pumped perfusate and the pulsation of the perfusate tank 100.

In this way, after an isolated heart perfusion apparatus operates normally, the heart 50 of the rat is isolated and is connected to the aortic cannula on a bottom end of the bubble trap 600 and then is plateaued for about 10 minutes and then, a perfusion flow rate (ml/min) is measured so that a normal heart state of the perfusion flow rate in the range of 10 to 28 ml/min can be checked.

As a result of checking, the supply of perfusate is blocked in a state in which the sensor unit 700 is inserted into or is in contact with the myocardium with respect to the heart 50 that maintains a normal function so that current signals corresponding to a gas concentration to be measured can be plateaued for about 30 minutes before ischemia occurs.

Subsequently, the perfusate to be continuously supplied is blocked using a valve on a bottom end of the heat exchanger 500 so that ischemia occurs, and after the ischemic state is maintained for a predetermined amount of time, the valve is opened so that the perfusate can be open.

In this case, it is checked using the oxygen sensor 720 that the oxygen concentration is reduced to '0' when ischemia occurs, and the control unit 900 analyzes its correlation with the degree of a myocardial injury using the oxygen concentration restored after reperfusion for 1 hour after ischemia occurs and the maximum oxygen concentration during reperfusion.

Also, a variation in NO concentration is observed in real time during an ischemic period using the NO sensor 740, and the control unit 900 analyzes the correlation with the degree of the myocardial injury using initial decrement and maximum increase of NO concentration while ischemia occurs and during reperfusion.

In this case, the oxygen sensor 720 and the NO sensor 740 are manufactured using a microsensor coated with a gas permeable layer and coil type Ag/AgCl reference electrodes.

The oxygen sensor 720 shows high sensitivity caused by a large effective area of the working electrode, and high selectivity due to low operation potential (-0.4 V vs. Ag/AgCl) and can continuously measure the changes in oxygen concentration within the myocardium in real time.

Also, the NO sensor 740 has high sensitivity due to surface treatment of working electrode and excellent selectivity with respect to NO due to NO permeable membrane, and can continuously measure a changes in NO concentration in the myocardium in real time.

### Induction of ischemia and reperfusion of heart 50 of experiment rat

As illustrated in FIG. 1, the heart 50 of the rat is connected to the biosignal measurement apparatus according to the present invention through cannulation of the aorta and is perfused by a K-H buffer solution under a continuous perfusion pressure of 70 mm Hg.

In this case, the perfusate is filed by a 5.0 µm microfilter, is bubbled together with 95% oxygen and 5% carbon dioxide at 37 °C so that pH 7.4 can be obtained.

Also, 37 °C is maintained by the double jacket storing tank and the heat exchanger through water circulation and is circulated in the entire apparatus using the peristaltic pump 200.

### Measurement of oxygen concentration in myocardium during ischemia and reperfusion of heart 50 of experiment rat

The oxygen sensor 720 treated with the gas permeable layer is inserted into the middle of the myocardium of the left ventricle of the isolated heart 50 of the rat so that a current is stabilized for 30 minutes before ischemia induction.

Ischemia initiates by stopping continuous perfusion, and reperfusion after ischemia induction is performed for 55 to 65 minutes, preferably, for 60 minutes.

The heart 50 of the rat is divided into five groups according to an ischemic period and is classified into a control group (0 minute), a first experiment group (10 minutes), a second experiment group (20 minutes), a third experiment group (30 minutes), and a fourth experiment group (40 minutes), and reperfusion is carried out for 60 minutes after ischemia induction.

### Variation in oxygen in myocardium during reperfusion after ischemia occurs

In order to investigate a variation in oxygen concentration (pO₂) in the reperfused myocardium after ischemia occurs for a predetermined time, a current variation caused by oxygen was monitored in real time in the heart 50 of the rat using the oxygen sensor 720 corrected by a current variation according to concentration. First, there was no changes in current measured by the oxygen sensor 720 during continuous perfusion for 130 minutes.

However, once ischemia initiates, the oxygen concentration (pO₂) is declined rapidly to near zero level and is maintained during ischemia. When reperfusion is initiated, a variation in the oxygen concentration (pO₂) depends on the induced ischemic period.

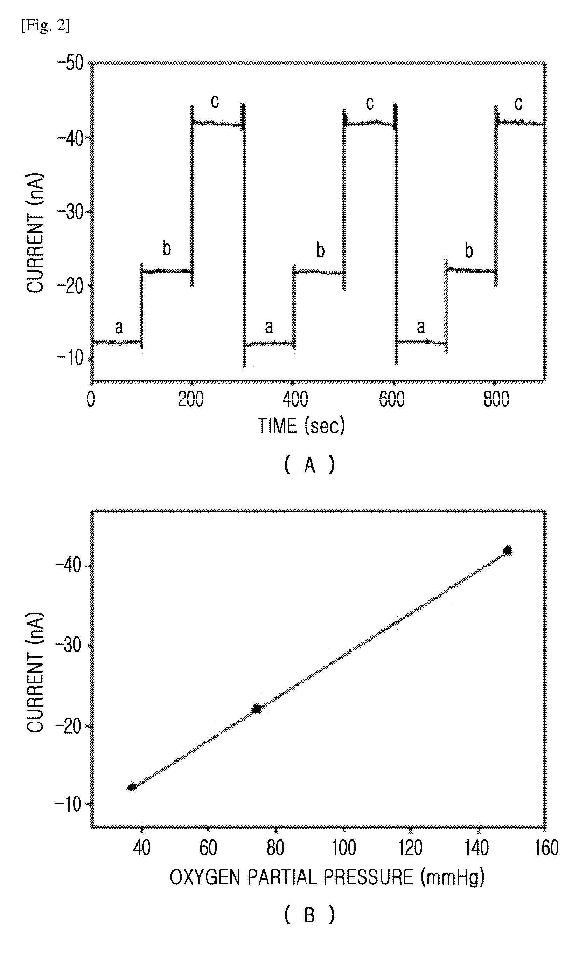

FIG. 2 is a graph showing representative patterns of an oxygen concentration (pO₂) response during cardiac ischemia reperfusion according to an ischemic period according to the present invention. (A) represents a first experiment group (10 minutes), and (B) represents a second experiment group (20 minutes), and (C) represents a third experiment group (30 minutes), and (D) represents a fourth experiment group (40 minutes).

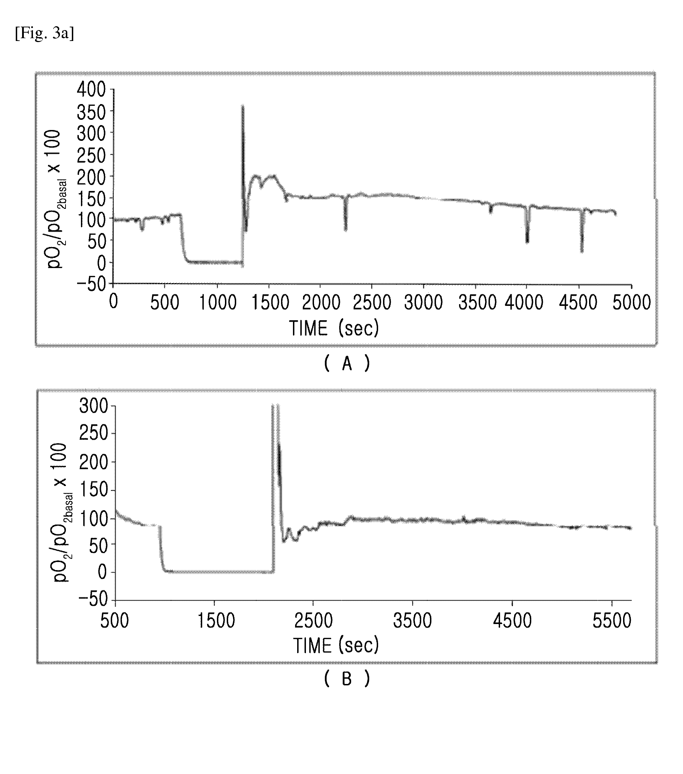

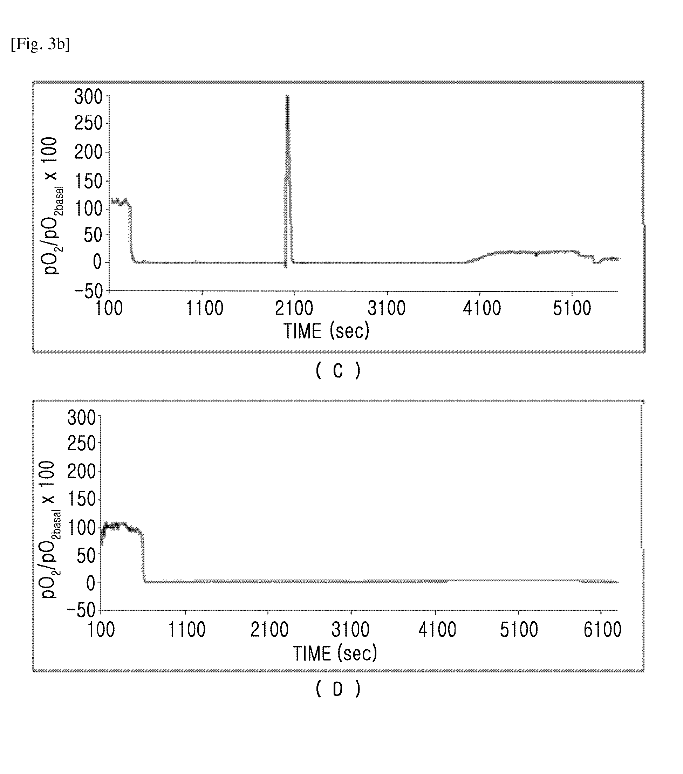

FIGS. 3A and 3B are graphs showing the definition of suggested analysis parameters with respect to a variation in the oxygen concentration according to an experimental protocol according to the present invention. FIGS. 3A and 3B show oxygen concentration (pO₂) dynamic 9 parameters for effectively analyzing the changes in the oxygen concentration (pO₂) after reperfusion of the first through fourth experiment groups initiates.

The change in the oxygen concentration in the myocardium during cardiac ischemia-reperfusion of the rat according to the definition of the parameters and the ischemic period is shown in Tables 1 and 2 below.

Table 1 shows the definition of parameters indicating the change in the oxygen concentration (pO₂) and an oxygen dynamic time used in the real-time biosignal measurement apparatus for cardiac ischemia and reperfusion according to the present invention.

Table 2 shows the changes in the oxygen concentration (pO₂) and an oxygen dynamic time according to a variation in the ischemic period in the real-time biosignal measurement apparatus for cardiac ischemia and reperfusion according to the present invention.

**[Table 1]**

| Parameters | | Definition |
|---|---|---|
| Changes in oxygen concentration (pO₂) | %pO₂ | Normalized pO₂ (pO₂/pO_{2 basal}) x 100 |
| | pO_{2basal} | Basal pO₂ during pre-ischemic period |
| | PO_{2isch} | pO₂ in ischemic plateau |
| | pO₂ᵣₑₚ₋ₘₐₓ | Maximum level of pO₂ during the reperfusion period |
| | pO₂ᵣₑₚ₋ᵣₑₛ | Restoration level of pO₂ after 60 min of reperfusion |
| Times for oxygen dynamic | Tᵢ₋ₚₗₐₜ | Time elapsed to reach ischemic plateau |
| | Tᵢ | Time of effective ischemia induction |
| | Tᵣₑₚ₋ₚₗₐₜ | Time to maintenance of ischemic plateau after initiation of reperfusion |
| | Tᵣₑₚ₋ₘₐₓ | Time of %pO₂ᵣₑₚ₋ₘₐₓ in reperfusion episode |
| | Tᵣₑₚ₋ᵣₑₛ | Time to maintenance of pO₂ᵣₑₚ₋ᵣₑₛ after Tᵣₑₚ₋ₘₐₓ |

**[Table 2]**

| Parameter | | Ischemic period | | | | P-value |
|---|---|---|---|---|---|---|
| | | 10min | 20min | 30min | 40min | |
| Oxygen concentration (pO₂) | %pO_{2-basal} | 98.25±5.11 | 98.73±3.96 | 99.49±0.54 | 98.68±2.03 | NS |
| | %pO_{2-ische} | 1.39±0.49 | 0.87±0.34 | 0.54±0.12 | 1.03±0.68 | NS |
| | %pO_{2-rep max} | 187.83±26.79 | 120.15±23.83 | 12.54±10.62 | 1.24±1.09 | < 0.001 |
| | %pO_{2-rep res} | 129.46±29.84 | 87.76±3.90 | 3.40±4.82 | 0.99±0.94 | < 0.001 |
| Time | Tᵢ₋ₚₗₐₜ | 79±42 | 87±37 | 158±138 | 49±35 | NS |
| | Tᵢ | 754±283 | 1113±37 | 1642±138 | 2351±35 | < 0.001 |
| | Tᵣₑₚ₋ₚₗₐₜ | NA | NA | 2714±1252 | * | NA |
| | Tᵣₑₚ₋ₘₐₓ | 851±1001 | 2265±1223 | 842±653 | NA | NS |
| | Tᵣₑₚ₋ᵣₑₛ | 2754±1006 | 1864±476 | 1892±1876 | NA | NS |

| | | | | | | |
|---|---|---|---|---|---|---|
| NA : non-applicable NS : insignificant | | | | | | |

As shown in Table 2, an ischemic plateau after the onset of ischemia was achieved within 79 ± 42 seconds in the first experiment group, 87 ± 37 seconds in the second experiment group, 158 ± 138 seconds in the third experiment group, and 49 ± 35 seconds in the fourth experiment group.

Also, there were no significant differences between the experiment groups in time Tᵢ₋ₚₗₐₜ that reaches the ischemic plateau and an oxygen concentration (pO₂) value.

On the other hand, once reperfusion initiates, in the first and second experiment groups, the oxygen concentration (pO₂) value was increased to a pre-ischemic level. However, in the third experiment group, the oxygen concentration (pO₂) value was increased to 2,714 ± 1,252 seconds, and in the fourth experiment group, the oxygen concentration (pO₂) value was not increased within a reperfusion period.

Also, the % oxygen concentration (pO₂) ᵣₑₚ₋ₘₐₓ value in the first experiment group was 187.83 ± 26.79, that in the second experiment group was 120.15 ± 23.83, that in the third experiment group was 12.54 ± 10.62, and that in the fourth experiment group was 1.24±1.09. Sixty minutes after the reperfusion, the % oxygen concentration (pO₂) ᵣₑₚₚᵣₑₛₛ value in the first experiment group was 129.46±29.84, that in the second experiment group was 87.76±3.90, that in the third experiment group was 3.40±4.82, and that in the fourth experiment group was 0.99±0.94.

FIG. 4 is a Box-and-Whisker graph showing maximum and restoration levels of the oxygen concentration during reperfusion that are percentages of pre-ischemic period levels according to the ischemic period according to the present invention.

As shown in FIG. 4, maximum and restoration values of the oxygen concentration (pO₂) of the third and fourth experiment groups after reperfusion were smaller than those of a pre-ischemic period, and the oxygen concentration (pO₂) value of the fourth experiment group was not restored.

To sum up, when ischemia was terminated within 20 minutes, the oxygen concentration (pO₂) in the myocardium was increased after the occurrence of reperfusion and was restored to a level of the pre-ischemic period. However, when the ischemic period exceeded 30 minutes, the oxygen concentration (pO₂) in the myocardium was not restored to the state of the pre-ischemic period.

Such a result shows that an ischemic time lasting >30 min causes a decrease in nutritive organ perfusion and the impairment of microcirculation during the reperfusion period.

### Variation in size of myocardial infarction according to ischemic period

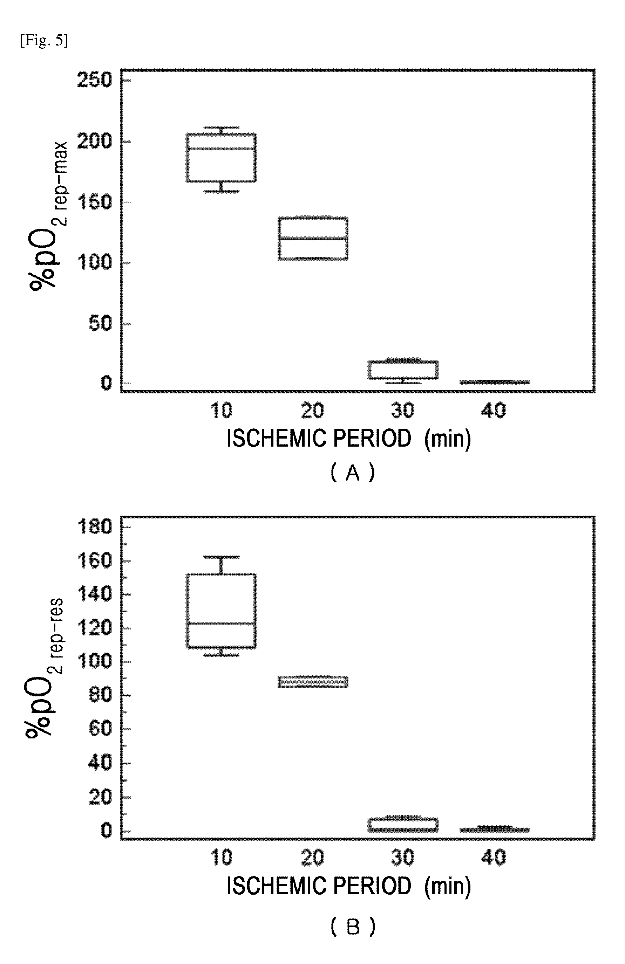

FIG. 5 is a graph showing the sizes of myocardial infarction of the rat according to a variation in the ischemic period according to the present invention.

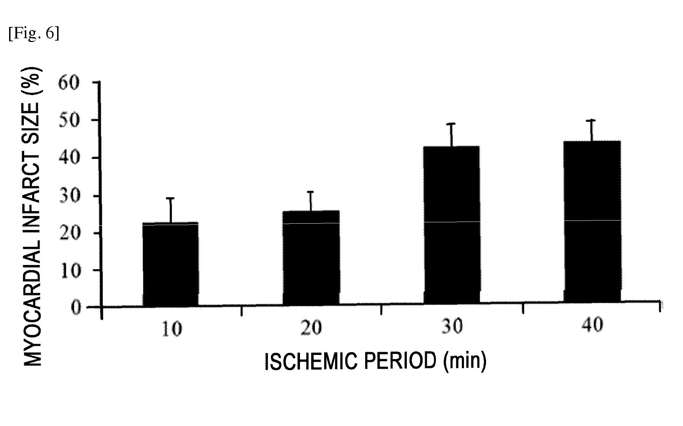

FIG. 6 are photos comparing the heart 50 of the rat, the sizes of the myocardial infarction changing according to a variation in the ischemic period according to the present invention, wherein the photos each represent (A) a normal state, (B) when the ischemic period is 10 minutes, (C) when the ischemic period is 20 minutes, (D) when the ischemic period is 30 minutes, and (E) when the ischemic period is 40 minutes.

The size of myocardial infarction is determined by a staining method with triphenyltetrazolium chloride (TTC). After 1 hour of reperfusion, the size of myocardial infarction is evaluated by staining with TTC, and myocardial infarction is indicated by a percentage of a risk territory.

As illustrated in FIG. 5, the sizes of myocardial infarction of the third and fourth experiment groups are 41.96 ± 6.23% and 43.02 ± 5.46% and are considerably larger than the size (22.57 ± 6.41%) of the first experiment group and the size (25.16 ± 5.08%) of the second experiment group. However, there was no large difference between the sizes of the third and fourth experiment groups.

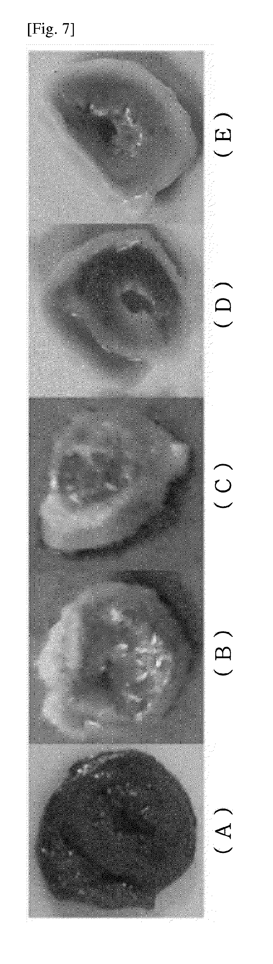

FIG. 7 is a graph showing the correlation between reoxygenation parameters of the myocardium after the ischemic period and the degree of severity of an ischemia-reperfusion (IR) injury according to the present invention.

As shown in FIG. 7, maximum and restoration values of the oxygen concentration (pO₂) of the myocardium after the occurrence of ischemia are closely related with the sizes of myocardial infarction.

That is, early microcirculation disorders are related with post-ischemic tissue damage, and oxygen supplied during this time of reperfusion with concurrent ventricular fibrillation is completely consumed due to either a high rate of oxidative phosphorylation or an arrhythmia-associated perfusion shunt.

Also, once arrhythmia is terminated, oxygenation of the heart is normalized, and contraction function is restored.

Thus, the degree of reoxygenation in post-ischemic myocardium is a significant index for an IR injury, myocardial viability, and postischemic cardiac dysfunction.

### Real-time simultaneous measurement of changes in oxygen and NO in myocardium during cardiac ischemia-reperfusion

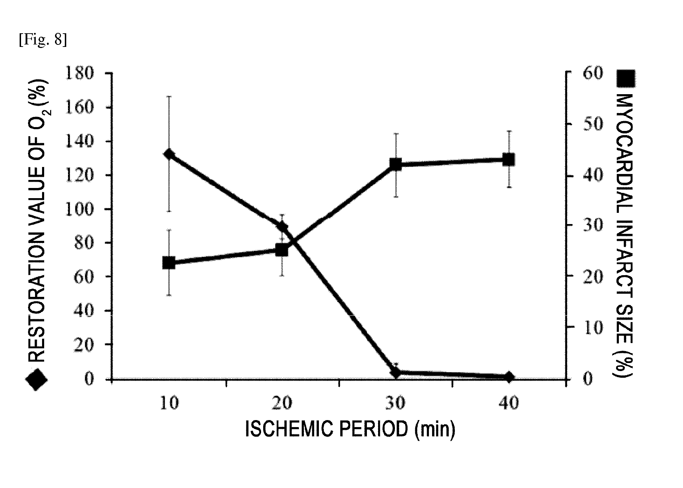

FIG. 8 is a graph showing the result of simultaneously measuring changes in nitric oxide (NO) and oxygen measured in the myocardium of rat during the ischemic period of 30 minutes and reperfusion for 60 minutes according to the present invention.

As shown in FIG. 8, an oxygen amount immediately after ischemic occurrence is rapidly reduced and reaches nearly a zero level and then is maintained during ischemia, whereas NO is slowly decreased and then is gradually increased from about 200 to 300 seconds and then is slightly reduced about 900 seconds.

Also, NO during reperfusion is rapidly increased and then is slowly decreased. On the other hand, oxygen is slightly increased when about 2000 seconds elapse after reperfusion is carried out.

This can be described by changes in NO synthase (NOS) expression observed during myocardial ischemia reperfusion. During ischemia, endothelial NOS (eNOS) activity increases within a few minutes, and subsequently the NO concentration increases during early ischemia. However, with prolonged myocardial ischemia, NOS3 protein expression decreases and the increased tissue acidosis attenuates eNOS activity, and the concentration of NO is reduced.

Thus, since synthesis of NO is closely related with oxygen, the correlation between the amount of endogenous NO release during ischemia and the degree of restoration of the oxygen concentration during reperfusion is checked and may be a significant base for determining the relationship between a change in endogenous NO and a myocardial protection effect.

In this way, a living heart state is maintained using the real-time biosignal measurement apparatus for cardiac ischemia and reperfusion according to the present invention, while checking whether cardiac ischemia occurs and reperfusion is performing normally using an oxygen sensor, and the variation of a endothelium dependent vasodilation gas molecule intrinsically generated in the myocardium during an ischemic period is measured so that oxygen and NO concentration in the myocardium can be continuously measured in real time using the oxygen sensor having a low operation potential and high sensitivity and an NO sensor having high sensitivity and high selectivity.

In addition, the correlation with a gasotransmitter concentration value of the myocardium of rat heart from a cardiac ischemia and reperfusion injury can be accurately analyzed, and the present invention can be usefully utilized to evaluate and select various drugs and treatments that may reinforce an endogenous protective mechanism against injury.

While the present invention has been particularly shown and described with reference to exemplary embodiments thereof, it will be understood that various changes in form and detail may be made therein without departing from the spirit and scope of the following claims.

## Claims

1. A real-time biosignal measurement apparatus for cardiac ischemia and reperfusion, comprising:
a peristaltic pump which pumps perfusate from a tank storing the perfusate;
a pressure trap which offsets the pulsation of both sides between a solution chamber temporarily storing the pumped perfusate and the perfusate tank;
a heat exchanger to which the temporarily stored perfusate is applied and which transfers the heat of the perfusate;
a bubble trap to which the heat transferred perfusate is applied on one side and which filters the heat transferred perfusate to transduce the perfusate to an isolated heart connected to the other side;
a sensor unit which detects the gas concentration value of the isolated heart in real time during the cardiac ischemia reperfusion; and
a control unit which is supplied with the gas concentration value of the isolated heart to analyze its correlation with the degree of a myocardial injury of the isolated heart.

2. The real-time biosignal measurement apparatus of claim 1, wherein the control unit analyzes the correlation using an oxygen concentration restored after reperfusion for a predetermined time after ischemia occurs and a maximum oxygen concentration during reperfusion.

3. The real-time biosignal measurement apparatus of claim 2, wherein the degree of a myocardial injury is evaluated by the size of myocardial infarction of the isolated heart according to a variation in an ischemic period.

4. The real-time biosignal measurement apparatus of claim 3, wherein the size of myocardial infarction is indicated by a percentage of a risk territory using a staining method with triphenyltetrazolium chloride (TTC).

5. The real-time biosignal measurement apparatus of claim 2, wherein the predetermined time is 55 to 65 minutes.

6. The real-time biosignal measurement apparatus of claim 1, wherein the sensor unit comprises at least one of:
an oxygen sensor that detects an oxygen concentration in the myocardium of the isolated heart in real time;
a nitric oxide (NO) sensor that detects an NO concentration in the myocardium of the isolated heart in real time;
a carbon oxide (CO) sensor that detects a CO concentration in the myocardium of the isolated heart in real time; and
a hydrogen sulfide sensor that detects a hydrogen sulfide concentration in the myocardium of the isolated heart in real time.

7. The real-time biosignal measurement apparatus of claim 6, wherein the sensor unit measures an oxygen concentration reduced when cardiac ischemia occurs, an oxygen concentration restored after reperfusion, and a maximum oxygen concentration during reperfusion using the oxygen sensor and measures a variation in the NO concentration during the ischemic period and initial decrement and maximum increment of NO concentration during ischemia and reperfusion using the nitric oxide sensor, thereby detecting the gas concentration value.

8. The real-time biosignal measurement apparatus of claim 1, further comprising a potentiostat that supplies a constant electric potential to the sensor unit and measures a change in current.

9. The real-time biosignal measurement apparatus of claim 1, wherein the heat exchanger comprises:
a thermocouple that is immediately in contact with a part to be connected to the aorta of the isolated heart; and
a temperature controller that maintains a temperature of the perfusate flowing into the isolated heart at a constant temperature.

10. The real-time biosignal measurement apparatus of claim 9, wherein the predetermined temperature is 35 °C to 39 °C .

11. The real-time biosignal measurement apparatus of claim 1, wherein the solution chamber maintains a predetermined height with the isolated heart.

12. The real-time biosignal measurement apparatus of claim 11, wherein the predetermined height is 85 to 95 cm.
